(19) Europäisches Patentamt
European Patent Office
Office européen des brevets

(11) Veröffentlichungsnummer: **0 501 317 A1**

## (12) EUROPÄISCHE PATENTANMELDUNG

(21) Anmeldenummer: **92102890.8**

(22) Anmeldetag: **21.02.92**

(51) Int. Cl.5: **B01J 23/66**, C07D 301/10

(30) Priorität: **01.03.91 DE 4106508**

(43) Veröffentlichungstag der Anmeldung:
**02.09.92 Patentblatt 92/36**

(84) Benannte Vertragsstaaten:
**BE DE FR GB NL**

(71) Anmelder: **BASF Aktiengesellschaft
Carl-Bosch-Strasse 38
W-6700 Ludwigshafen(DE)**

(72) Erfinder: **Herzog, Klaus, Dr.
Oggersheimer Strasse 112
W-6700 Ludwigshafen(DE)**
Erfinder: **Boehning, Karl-Heinz, Dr.
Bruesseler Ring 32
W-6700 Ludwigshafen(DE)**
Erfinder: **Plueckhan, Juergen, Dr.
Bensheimer Ring 19 b
W-6710 Frankenthal(DE)**
Erfinder: **Mross, Wolf Dieter, Dr.
Anselm-Feuerbach-Strasse 21
W-6710 Frankenthal(DE)**
Erfinder: **Schwarzmann, Matthias, Dr.
Carl-Bosch-Strasse 54
W-6703 Limburgerhof(DE)**

(54) **Silberkatalysator.**

(57) Silberkatalysator mit Lithium und Cäsium als Promotor für die Direktoxidation von Ethylen mit Sauerstoff zu Ethylenoxid auf einem porösen Träger, der im wesentlichen aus $\alpha$-Aluminiumoxid besteht und bestimmte Mengen an salpetersäurelöslichen Calcium-, Aluminium-, Kalium- und Natriumverbindungen enthält, dessen Silbergehalt mindestens 14 Gew.-%, dessen Cäsium- und/oder Rubidiumgehalt 0,05-0,2 Gew.-% und dessen Lithiumgehalt 0,025-0,4 Gew.-%, bezogen jeweils auf den gesamten Katalysator, beträgt und dessen Träger eine BET-Oberfläche von 1-2 $m^2/g$ und ein Porenvolumen von 0,40-0,55 ml/g hat und salpetersäurelösliche Verbindungen von maximal 400 Gew.-ppm Aluminium, 300 Gew.-ppm Calcium, 150 Gew.-ppm Kalium und 150 Gew.-ppm Natrium enthält sowie ein Verfahren zu dessen Herstellung und die Verwendung dieses Katalysators zur Herstellung von Ethylenoxid aus Ethlyen und Sauerstoff in der Gasphase.

EP 0 501 317 A1

Die vorliegende Befindung betrifft einen Silberkatalysator, das Verfahren zu seiner Herstellung sowie seine Verwendung als Katalysator zur Herstellung von Ethylenoxid aus Ethylen und Sauerstoff.

Für die Herstellung moderner Katalysatoren für die Direktoxidation von Ethylen mit Sauerstoff zu Ethylenoxid werden derzeit als Trägermaterial ausschließlich $\alpha$-Aluminiumoxide verwendet.

Die in vielen Katalysatorpatenten angegebenen wünschenswerten Eigenschaften, die das Trägermaterial aufweisen soll, beschränken sich zumeist auf Aussagen über den Gehalt an $\alpha$-Aluminiumoxid, BET-Oberfläche, Porendurchmesser, Porenvolumen/Porosität und Schüttgewicht. Besonderer Wert wird im allgemeinen auf die enge Eingrenzung der BET-Oberfläche der Träger gelegt, wobei als besonders bevorzugt Werte von unter 1 $m^2/g$ genannt werden.

So werden in DE-A 30 10 533 Oberflächen von weniger als 1 $m^2/g$ und Porenvolumina von 0,2 bis 0,6 $cm^3/g$ bei einem Porendurchmesser zwischen 500 - 50000 nm angegeben. Die chemische Zusammensetzung des Trägers wird in dieser Schrift als nicht entscheidend bezeichnet.

In DE-A 25 21 906 werden $\alpha$-Aluminiumoxidträger mit BET-Oberflächen von 0,1 bis 2 $m^2/g$ als besonders geeignet beschrieben. Als besonders vorteilhaft werden hier Träger mit BET-Oberflächen von 0,1 - 0,8 $m^2/g$ angesehen, da an Katalysatoren auf Trägern mit BET-Oberflächen von 1,07-6,67 $m^2/g$ im Vergleich zu Trägern mit BET-Oberflächen von 0,17-0,35 $m^2/g$ um 2-5 Prozentpunkte geringere Selektivitäten erzielt werden.

In EP-A 211 397 und EP-A 271 814 wurde erstmals festgestellt, daß der Gehalt des Trägermaterials an salpetersäurelöslichen Calcium-, Aluminium-, Kalium- und Natriumionen die Selektivität des Katalysators beeinflußt. Doch auch nach der Lehre dieser Schriften werden Katalysatoren mit BET-Oberflächen von weniger als 1 $m^2/g$, nämlich mit 0,4-0,8 $m^2/g$ angewandt.

Da für die Katalysatoren zur Herstellung des großtechnischen Produktes Ethylenoxid ein ständiger Verbesserungsbedarf besteht, lag der vorliegenden Erfindung die Aufgabe zugrunde, verbesserte Katalysatoren zur Herstellung von Ethylenoxid zur Verfügung zu stellen.

Dementsprechend wurde ein Silberkatalysator mit Lithium und Cäsium als Promotor für die Direktoxidation von Ethylen mit Sauerstoff zu Ethylenoxid auf einem porösen Träger, der im wesentlichen aus $\alpha$-Aluminiumoxid besteht und bestimmte Mengen an salpetersäurelöslichen Calcium-, Aluminium-, Kalium- und Natriumverbindungen enthält, gefunden, der dadurch gekennzeichnet ist, daß sein Silbergehalt mindestens 14 Gew.-%, sein Cäsium- und/oder Rubidiumgehalt 0,05-0,2 Gew.-% und sein Lithiumgehalt 0,025-0,4 Gew.-%, bezogen jeweils auf den gesamten Katalysator, beträgt, der für den Katalysator verwendete Träger eine BET-Oberfläche von 1-2 $m^2/g$ und ein Porenvolumen von 0,40-0,55 ml/g hat und salpetersäurelösliche Verbindungen von max. 400 Gew.-ppm Aluminium, 300 Gew.-ppm Kalzium, 150 Gew.-ppm Kalium und 150 Gew.-ppm Natrium enthält.

Des weiteren wurde ein Verfahren zur Herstellung des obengenannten Silberkatalysators gefunden, das dadurch gekennzeichnet ist, daß man einen $\alpha$-Aluminiumoxidträger mit einer BET-Oberfläche von 1-2 $m^2/g$, einem Porenvolumen von 0,40-0,55 ml/g und einen Gehalt an salpetersäurelöslichen Verbindungen von max. 400 Gew.-ppm Aluminium, 300 Gew.-ppm Calcium, 150 Gew.-ppm Kalium und 150 Gew.-ppm Natrium mit einer Silbersalzlösung, welche komplexbildende Zusätze enthält und mit Lösungen der Alkalimetallpromotoren Lithium und Cäsium und/oder Rubidium in ein oder zwei Stufen imprägniert und tempert, so daß der fertige Katalysator mindestens 14 Gew.-% Silber, 0,025-0,4 Gew.-% Lithium und 0,05-0,2 Gew.% Cäsium und/oder Rubidium enthält. Des weiteren betrifft die Erfindung die Verwendung des obengenannten Katalysators zur Herstellung von Ethylenoxid aus Ethylen und Sauerstoff in der Gasphase.

Der für die Wirksamkeit der erfindungsgemäßen Katalysatoren wichtige Katalysatorträger wird aus Aluminiumoxid hergestellt. Sein Gehalt an $\alpha$-Aluminiumoxid beträgt vorzugsweise mehr als 99 Gew.-%. Der Gehalt dieses Trägermaterials an Aluminium-, Calcium-, Kalium- und Natriumverbindungen, die sich durch Kochen mit verdünnter Salpetersäure auswaschen lassen, soll einen Gehalt von 400 Gew.-ppm Aluminium, 300 Gew.-ppm Calcium, 150 Gew.-ppm Kalium und 150 Gew.-ppm Natrium nicht übersteigen. Dieses hochreine Trägermaterial soll eine BET-Oberfläche von nicht weniger als 1 $m^2/g$, vorzugsweise eine BET-Oberfläche von 1-2 $m^2/g$ und besonders bevorzugt von 1,5-2 $m^2/g$ haben. Das Porenvolumen dieses Trägermaterials sollte mindestens 0,4 $cm^3/g$ betragen. Bevorzugt werden Trägermaterialien mit Porenvolumina von 0,4-0,55 $cm^3/g$ eingesetzt. Das Porenvolumen des Trägermaterials kann zweckmäßigerweise durch die allgemein bekannten Methoden der Bestimmung der Wasseraufnahme oder der Quecksilberporosimetrie bestimmt werden. Bei der Bestimmung des Porenvolumens über die Ermittlung de Wasseraufnahme soll sich das durch 5 minütiges Einwirken von Wasser bei Raumtemperatur festgestellte Porenvolumen nur unwesentlich, max. 10 %, von dem mit Hilfe der Quecksilberporosimetrie gemessenen Volumen unterscheiden.

Die Porenverteilung kann unimodal oder bimodal sein, wobei im Falle einer bimodalen Verteilung die größeren Poren vorzugsweise 50 % des Gesamtporenvolumens ergeben und einen mittleren Porendurchmesser von etwa 10 - 50 $\mu$m haben. Die kleineren Poren haben im Falle einer bimodalen Verteilung vorzugsweise einen mittleren Porendurchmesser von 0,5-2 $\mu$m und bei einer unimodalen Verteilung von 1-5 $\mu$m.

Es wurde gefunden, daß sich besonders vorteilhafte Silberkatalysatoren zur Herstellung von Ethylenoxid durch die Direktoxidation von Ethen aus dem obenbeschriebenen Trägermaterial herstellen lassen, wenn diese bei einer Silberbeladung von mindestens 14 Gew.-%, insbesondere bei einer Silberbeladung zwischen 14 und 20 Gew.-% mit 0,05-0,2, vorzugsweise 0,1-0,2 Gew.-% Cäsium und/oder Rubidium und mit 0,025-0,4 Gew.-% Lithium, jeweils bezogen auf das Gesamtgewicht des Katalysators, dotiert sind.

Die erfindungsgemäßen Silberkatalysatoren können aus dem genannten Trägermaterial in ein oder zwei Stufen hergestellt werden. Bei einer einstufigen Herstellung des Katalysators werden die Aktivkomponente Silber und die Alkalimetallpromotoren Lithium und Cäsium simultan in einer Tränkstufe auf den Katalysatorträger aufgebracht. Bei der zweistufigen Herstellung des Silberkatalysators werden in der 1. Stufe das Silber und der Promotor Lithium und in der 2. Stufe das schwere Alkalimetall Cäsium auf den Katalysatorträger aufgetragen. Anstelle des schweren Alkalimetallpromotors Cäsium kann mit praktisch gleicher Wirksamkeit das schwere Alkalimetall Rubidium verwendet werden. Zur Aufbringung der Aktivkomponente auf das Trägermaterial werden zweckmäßigerweise mit Komplexbildnern, vorzugsweise mit Aminen, stabilisierte Silbersalzlösungen, vorzugsweise Silbernitratlösungen, eingesetzt. Für diesen Zweck geeignete Komplexbildner sind beispielsweise in DE-A 25 21 906 beschrieben, auf die hiermit Bezug genommen wird. Nach der Imprägnierung mit den Lösungen der Aktivkomponente Silber und den Lösungen der Promotoren Lithium und Cäsium und/oder Rubidium wird der imprägnierte Katalysatorträger in der Regel, je nachdem ob das ein- oder das zweistufige Verfahren zur Herstellung des Silberkatalysators angewandt wird, bei 180 - 300°C ein- oder zweimal getempert.

Die auf diese Weise erhältlichen Silberkatalysatoren werden zur Herstellung von Ethylenoxid durch die Direktoxidation von Ethylen mit Sauerstoff in der Gasphase verwendet. Bei der Herstellung von Ethylenoxid mit Hilfe des erfindungsgemäßen Silberkatalysators kann dabei unter an für sich herkömmlichen Reaktionsbedingungen gearbeitet werden, wie sie beispielsweise in DE-A 25 21 906 beschrieben sind, auf die hiermit Bezug genommen wird.

Die erfindungsgemäßen Katalysatoren sind gegenüber den Katalysatoren des Standes der Technik selektiver und aktiver und haben außerdem eine verbesserte Stabilität, welche eine Verlängerung der Standzeit zur Folge hat.

Beispiele:

Herstellung der Katalysatoren

Die im folgenden aufgeführten, erfindungsgemäßen und nicht erfindungsgemäßen Katalysatoren mit den verschiedenen Trägern wurden auf folgende Weise hergestellt:
Silbernitrat wurde in der zweifach molaren Menge s-Butylamin zum Silber-s-Butylamin-Komplex umgesetzt. Dieser Lösung wurde die jeweils betreffende Menge an Lithiumnitrat in Form einer wäßrigen Lösung zugesetzt.

Entsprechend der zu erwartenden Flüssigkeitsaufnahme der Katalysatorträger wurden diese mit der erhaltenen Lösung imprägniert und anschließend drei Stunden bei Raumtemperatur gelagert. Die imprägnierten Träger wurden dann in einem Umluftofen bei einer Temperatur von 240°C bis zur vollständigen Zersetzung des Silberkomplexes getempert.

Die so erhaltenen Grundkatalysatoren wurden mit einer wäßrigen Cäsiumnitratlösung imprägniert, welche zusätzlich neben der betreffenden Menge an Cäsiumnitrat noch die, bezogen auf die vorher aufgebrachte Silbermenge, zweifach molare Menge an s-Butylamin und Ammoniumnitrat enthielt. Die so imprägnierten Katalysatorträger wurden drei Stunden bei Raumtemperatur gelagert und anschließend in einem Umluftofen unter einem Stickstoffstrom getempert, wobei die Temperatur im Umluftofen jeweils so gewählt wurde, daß die Zersetzung des Ammoniumnitrats nach 20 min beendet war. Diese Temperatur wurde für die einzelnen Träger in Vorversuchen ermittelt.

Die Eigenschaften der für die erfindungsgemäßen (A,B) und nichterfindungsgemäßen (C,D,E) Silberkatalysatoren verwendeten Trägermaterialien sind in Tabelle 1 zusammengestellt.

Tabelle 1

|  |  | A | B | C | D | E |
|---|---|---|---|---|---|---|
| Gehalt an Si | ( Gew.%) | 0,21 | 0,28 | 0,42 | 0,21 | 0,13 |
| lösliche Ionen |  |  |  |  |  |  |
| Al | (Gew.-ppm) | 260 | 470 | 2200 | 700 | 1200 |
| Ca | (Gew.-ppm) | 50 | 170 | 700 | 140 | 500 |
| K | (Gew.-ppm) | 10 | 100 | 596 | 20 | 25 |
| Na | (Gew.-ppm) | 150 | 140 | 438 | 380 | 1800 |
| BET-Oberfläche | ( m2/g ) | 1,00 | 1,83 | 0,76 | 0,72 | 2,1 |
| Wasseraufnahme kalt (20 °C) | ( ml/g ) | 0,41 | 0,46 | 0,53 | 0,41 | 0,24 |
| Porendurchmesser | ( μm ) |  |  |  |  |  |
| erste- |  | 3,1 | 18,0 | 25,0 | 3,2 | 0,7 |
| zweite Verteilung |  | - | 0,5 | 0,5 | - | - |
| Abrieb | ( Gew.-% ) | 2,1 | 1,3 | 9,0 | 1,7 | 0,1 |

Die Bestimmung des Gehaltes an löslichen Ionen wurde so durchgeführt, daß man ca. 10 g Trägerpartikel 10 min mit halbkonzentrierter Salpetersäure kochte und die filtrierte Lösung des so erhaltenen Extraktes zur quantitativen Bestimmung der betreffenden Elemente durch Atomabsorptions-spektrometrie verwendete.

Herstellung von Ethylenoxid

Die gemäß dem obenbeschriebenen Verfahren hergestellten Silberkatalysatoren wurden zerkleinert und gesiebt. Jeweils 10 g der Siebfraktion von 0,6-0,75 mm Durchmesser wurden in einen Reaktor aus rostfreiem Stahl mit einem Innendurchmesser von 5 mm gefüllt. Der Reaktor wurde mittels eines Mantels, durch den eine Heizflüssigkeit geleitet wurde, auf die gewünschte Reaktionstemperatur thermostatisiert. Durch den Reaktor wurde ein Gas der Zusammensetzung:
30 Vol.-% Ethylen
8 Vol.-% Sauerstoff
2 ppm Vinylchlorid (Inhibitor)
Rest Stickstoff
Die Ergebnisse dieser Versuche sind in den Tabellen 2-5 aufgeführt.

Tabelle 2:

Katalysatoren auf Träger A, Silbergehalt: 14 Gew.-%,
Lithiumgehalt: 250 Gew.-ppm

| Katalysator | Cäsiumgehalt [Gew.-ppm] | Aktivität [°C] | Selektivität [%] |
|---|---|---|---|
| A1 | 500 | 220 | 81,7 |
| A2 | 600 | 220 | 82,3 |
| A3 | 700 | 222 | 81,5 |
| A4 | 800 | 228 | 80,6 |
| A5 | 900 | 265 | 73,7 |

Tabelle 3

Katalysatoren auf Träger B, Silbergehalt: 16 Gew.-%,
Lithiumgehalt: 250 Gew.-ppm

| Katalysator | Cäsiumgehalt [Gew.-ppm] | Aktivität [°C] | Selektivität [%] |
|---|---|---|---|
| B1 | 600 | 197 | 80,2 |
| B2 | 800 | 202 | 81,5 |
| B3 | 1000 | 206 | 82,4 |
| B4 | 1200 | 210 | 82,7 |
| B5 | 1400 | 213 | 82,1 |
| B6 | 1600 | 225 | 80,8 |

Tabelle 4

Katalysatoren auf Träger C, Silbergehalt: 18 Gew.%,
Lithiumgehalt: 250 Gew.-ppm

| Katalysator | Cäsiumgehalt [Gew.-ppm] | Aktivität [°C] | Selektivität [%] |
|---|---|---|---|
| C1 | 250 | 223 | 80,5 |
| C2 | 300 | 226 | 81,0 |
| C3 | 350 | 226 | 81,2 |
| C4 | 400 | 226 | 81,7 |
| C5 | 450 | 228 | 81,7 |
| C6 | 500 | 231 | 81,0 |

Tabelle 5

Katalysatoren auf Träger D, Silbergehalt: 14 Gew.-%,
Lithiumgehalt: 250 Gew.-ppm

| Katalysator | Cäsiumgehalt [Gew.-ppm] | Aktivität [°C] | Selektivität [%] |
|---|---|---|---|
| D1 | 250 | 221 | 80,5 |
| D2 | 300 | 223 | 81,2 |
| D3 | 350 | 223 | 81,8 |
| D4 | 400 | 224 | 81,4 |
| D5 | 450 | 228 | 80,3 |

Tabelle 6

Katalysatoren auf Träger E, Silbergehalt: 10,1 Gew.-%,
Lithiumgehalt: 250 Gew.-ppm

| Katalysator | Cäsiumgehalt [Gew.-ppm] | Aktivität [°C] | Selektivität [%] |
|---|---|---|---|
| E1 | 0 | 217 | 71,0 |
| E2 | 200 | 221 | 75,6 |
| E3 | 400 | 229 | 76,6 |
| E4 | 600 | 234 | 75,9 |
| E5 | 800 | 235 | 75,5 |
| E6 | 1000 | 239 | 72,2 |

**Patentansprüche**

1. Silberkatalysator mit Lithium und Cäsium als Promotor für die Direktoxidation von Ethylen mit Sauerstoff zu Ethylenoxid auf einem porösen Träger, der im wesentlichen aus α-Aluminiumoxid besteht und bestimmte Mengen an salpetersäurelöslichen Calcium-, Aluminium-, Kalium- und Natriumverbindungen enthält, dadurch gekennzeichnet, daß sein Silbergehalt mindestens 14 Gew.-%, sein Cäsium- und/oder Rubidiumgehalt 0,05-0,2 Gew.-% und sein Lithiumgehalt 0,025-0,4 Gew.-%, bezogen jeweils auf den gesamten Katalysator, beträgt, der für den Katalysator verwendete Träger eine BET-Oberfläche von 1-2 $m^2$/g und ein Porenvolumen von 0,40-0,55 ml/g hat und salpetersäurelösliche Verbindungen von maximal 400 Gew.-ppm Aluminium, 300 Gew.-ppm Calcium, 150 Gew.-ppm Kalium und 150 Gew.-ppm Natrium enthält.

2. Verfahren zur Herstellung eines Silberkatalysators gemäß Anspruch 1, dadurch gekennzeichnet, daß man einen $\alpha$-Aluminiumoxidträger mit einer BET-Oberfläche von 1-2 $m^2$/g, einem Porenvolumen von 0,40-0,55 ml/g und einem Gehalt an salpetersäurelöslichen Verbindungen von max. 400 Gew.-ppm Aluminium, 300 Gew.-ppm Calcium, 150 Gew.-ppm Kalium und 150 Gew.-ppm Natrium mit einer Silbersalzlösung, welche komplexbildende Zusätze enthält und mit Lösungen der Alkalimetallpromotoren Lithium und Cäsium und/oder Rubidium in ein oder zwei Stufen imprägniert und tempert, so daß der fertige Katalysator mindestens 14 Gew.-% Silber, 0,025-0,4 Gew.-% Lithium und 0,05-0,2 Gew.-% Cäsium und/oder Rubidium enthält.

3. Verwendung des Silberkatalysators gemäß Anspruch 1 zur Herstellung von Ethylenoxid aus Ethylen und Sauerstoff in der Gasphase.

Europäisches
Patentamt

# EUROPÄISCHER RECHERCHENBERICHT

Nummer der Anmeldung

EP 92 10 2890

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 327 356 (CHINA PETROCHEMICAL CORP.) * Seite 1, Zeilen 33-39; Seite 2, Zeilen 33-40; Seite 5, Zeilen 1-17 * --- | 1-3 | B 01 J 23/66 C 07 D 301/10 |
| A | EP-A-0 247 414 (MITSUBISHI PETROCHEMICAL CO.) * Seite 8, Zeilen 1-4,33-35 * --- | 1-3 | |
| X,A | US-A-4 368 144 (MASASHI MITSUHATA et al.) * Spalte 5, Zeilen 52-68; Spalte 6, Zeilen 1-28 * --- | 1-3 | |
| A,D | EP-A-0 271 814 (BASF) * Seite 2, Zeilen 22-32; Seite 4, Zeilen 15-45 * --- | 1-3 | |
| A,D | EP-A-0 211 397 (BASF) * Seite 2, linke Spalte, Zeilen 49-55, rechte Spalte, Zeilen 1-13; Seite 3, linke Spalte, Zeilen 1-20; Seite 4, Tabelle * ----- | 1-3 | **RECHERCHIERTE SACHGEBIETE (Int. Cl.5)** B 01 J C 07 D |

Der vorliegende Recherchenbericht wurde für alle Patentansprüche erstellt

| Recherchenort | Abschlußdatum der Recherche | Prüfer |
|---|---|---|
| DEN HAAG | 25-05-1992 | KERRES P.M.G. |